(19) Europäisches Patentamt
European Patent Office
Office européen des brevets

(11) Publication number: **0 285 883**
**A2**

(12) # EUROPEAN PATENT APPLICATION

(21) Application number: **88104363.2**

(51) Int. Cl.4: **A61K 31/16**

(22) Date of filing: **18.03.88**

The microorganism has been deposited with the Fermentation Research Institute under number Ferm - P5083.

The title of the invention has been amended (Guidelines for Examination in the EPO, A-III, 7.3).

(30) Priority: **19.03.87 JP 62689/87**

(43) Date of publication of application: **12.10.88 Bulletin 88/41**

(84) Designated Contracting States: **DE FR GB IT**

(71) Applicant: **ZAIDAN HOJIN BISEIBUTSU KAGAKU KENKYU KAI**
**14-23, Kamiosaki 3-chome**
**Shinagawa-ku Tokyo(JP)**

(72) Inventor: **Aoyagi, Takaaki**
**3-6, Hon Kugenuma 3-chome**
**Fujisawa-shi Kanagawa-ken(JP)**
Inventor: **Tabira, Takeshi**
**I-201, Ogawa Higashi-cho 4-1-1**
**Kodaira-shi Tokyo(JP)**
Inventor: **Takeuchi, Tomio**
**1-11, Higashi Gotanda 5-chome**
**Shinagawa-ku**
**Tokyo(JP)**

(74) Representative: **Lederer, Franz, Dr. et al**
**Van der Werth, Lederer & Riederer**
**Patentanwälte Lucile-Grahn-Strasse 22**
**D-8000 München 80(DE)**

(54) **Bactobolin as a prophylactic or therapeutic agent for autoimmune diseases, and as an immuno-suppressive agent.**

(57) A known antibiotic substance, bactobolin is now found to be useful to treat an autoimmune disease, as demonstrated by the treatment of experimental allergic encephalomyelitis (EAE) with bactobolin. Bactobolin is also effective to suppress lymphocyte proliferative response to specific antigen or mitogen. Generally, bactobolin is now found to be effective as an immunosuppressive agent.

EP 0 285 883 A2

Xerox Copy Centre

# Prophylactic or Therapeutic Agent for Autoimmune Diseases, and Immunosuppressive Agent

This invention relates to a new prophylactic or therapeutic agent for an autoimmune disease such as multiple sclerosis, acute disseminated encephalomyelitis and collagen disease, which comprises bactobolin as the active ingredient. This invention also relates to new use of bactobolin as a prophylactic or therapeutic agent for the autoimmune disease or as an immunosuppressive agent which is useful to suppress the immune response in a living animal, including human. Further, this invention relates to a method of prophylactically or therapeutically treating an autoimmune disease, and besides it relates to a method for suppressing the immune response in a living animal, inclusive of human, in particular, for suppressing abnormal immune response which would occur in a living animal as invoked by an autoimmune disease.

Furthermore, this invention relates to a method for prophylactically or therapeutically treating an experimental allergic encephalomyelitis (EAE), in the induction phase or in the effector phase, which comprises administering an effective amount of bactobolin to a living animal having suffered from the experimental allergic encephalomyelitis as induced. Particularly, this invention relates to a method for suppressing lymphocyte proliferative response to specific antigen or mitogen, which comprises administering an effective amount of bactobolin to a living animal, including human.

## BACKGROUND OF THE INVENTION

As the conventional prophylactic or therapeutic agents for autoimmune diseases, there are known anti-lymphocyte immuno-globulin, glucocorticoid preparations, nucleic acid metabolic antagonists, alkylating agents and the like.

Many of the above-mentioned conventional prophylactic or therapeutic agents for autoimmune diseases are, however, dissatisfactory in their effectiveness and toxicity, and better drugs have long been awaited. It is an object of this invention to meet such a long standing demand.

## DETAILED DESCRIPTION OF THE INVENTION

We, the present inventors, have now found that a known antifiotic, bactobolin represented by the following formula (I):

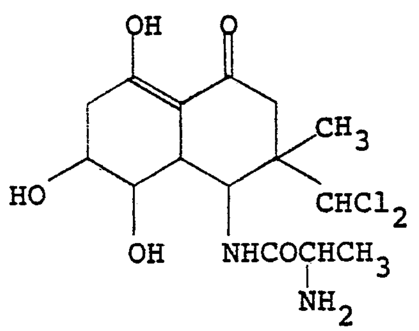

(I)

show prophylactic or therapeutic effects on the autoimmune diseases.

Bactobolin which is useful in the practice of this invention is a known antibiotic compound as produced by a bacterial strain of the genus Pseudomonas and is known to have antibacterial activities and antitumor activities {Japanese Patent Application first publication "Kokai" No. 22777/81; Japanese patent No. 1,414,049; and the "Journal of Antibiotics" Vol. 32, page 1069(1979)}. This invention is based on our unexpected discovery that bactobolin exhibits prophylactic or therapeutic effects on autoimmune diseases. These prophylactic or therapeutic effects of bactobolin (hereinafter sometime called "the present substance") on autoimmune diseases can be demonstrated by the animal tests given hereinafter. The present substance, bactobolin having such medical effects is now found to be useful as a prophylactic or therapeutic agent for autoimmune diseases, such as multiple sclerosis, acute disseminated encephalomyelitis and collagen disease. According to Japanese Patent Application first publication "Kokai"

No. 22777 81, the acute $LD_{50}$ toxicity of bactobolin is 6.25-12.5 mg/kg (in mouse, intravenous).

Thus, from the animal tests using rats which was caused to develop EAE by transferring the EAE-causing agents, specifically we have now found that the administration of bectobolin is effective to suppress or reduce the number of incidence of EAE and also suppress or reduce the histological changes in some separate sections of the EAE rats. Besides, we have found that the administration of bactobolin to rats is effective to reduce the degree of lymphocyte proliferative response to specific antigen or mitogen. It can therefore be concluded that bactobolin is active to suppress the immune response in a living animal, including human.

According to a first aspect of this invention, therefore, there is provided a prophylactic or therapeutic agent for an autoimmune disease, which comprises bactoboline as the active ingredient, in association with a pharmaceutically acceptable carrier for the active ingredient.

According to a second aspect of this invention, there is provided a method of prophylactically or therapeutically treating an autoimmune disease, which comprises administering an effective amount of bactobolin to an animal having suffered from the autoimmune disease.

According to another aspect of this invention, there is provided a new use of bactobolin as an immunosuppressive agent to suppress the immune response in a living animal, including human.

According to still another aspect of this invention, there is provided a method of suppressing the immune response in a living animal, which comprises administering an effective amount of bactobolin to a living animal where the immune response are to be suppressed.

According to a further aspect of this invention, there is provided a method for suppressing prophylactically or therepeutically treating an experimental allergic encephalomyelitis in the induction phase or in the effector phase, which comprises administering an effective amount of bactobolin to an animal having suffered from the experimental allergic encephalomyelitis as induced.

According to a particular aspect of this invention, there is provided a method for suppressing lymphocyte proliferative response to antigen or mitogen, which comprises administering an effective amount of bactobolin to living animal where the lymphocyte proliferative response to antigen or mitogen is to be suppressed.

When bactoboline is administered, bactobolin may be given also in the form of its acid addition salt with a pharmaceutically acceptable acid such as hydrochloric acid, sulfuric acid, phosphoric acid, acetic acid, citric acid, malic acid, methanesulfonic acid and the like.

Bactobolin which is used in the practice of the present invention can be produced, for example, by a fermentative method as described in Referential Examples 1 and 2 given hereinafter. In order to utilize bactobolin as the active ingredient in a prophylactic or therapeutic agent for autoimmune diseases, it may be used in the form of a free base or a salt with a non-toxic acid such as hydrochloric acid, salfuric acid and acetic acid.

The present agent containing bactobolin as the active ingredient may be formulated into various preparation forms, for example, injections such as intramuscular injections and intravenous injections, oral preparations such as capsules, tablets, granules, powder and liquid, and external preparations such as ointment and rectal preparations, in association with a pharmaceutically acceptable solid or liquid carrier. Although the dose of bactobolin varies depending on the administration route, preparation form, symptom of disease, etc., it is preferable to choose the dosage in a range of 1-1,000 mg for an adult per day.

Bactobolin may be formulated into a dosage unit form by a usual method. Various excipients as known in the pharmaceutical technology of drug preparation may also be mixed with bactobolin so long as they do not give any side adverse effects on the present substance. To formulate bactobolin into an injectable solution, the bactobolin may be dissolved in injection-grade distilled water or physiological saline, and after addition of a stabilizer, dispersant, soothing agent and/or the like as needed, the resultant solution is filled in injection ampules. As an alternative, the solution of bactobolin is lyophilized in a vial and upon administration, it is again dissolved in injection-grade distilled water or physiological saline. In order to formulate bactobolin into an oral preparation, bactobolin may be added and mixed with an excipient, stabilizer, antiseptic, dispersant and the like as known in the art and is then formulated into a suitable oral preparation form. In order to formulate bactobolin into an external preparation, bactobolin may be added and mixed with a substrate, stabilizer, antiseptic, surfactant and the like and is then formulated into a suitable external preparation form.

Production of bactobolin by the fermentative method will next be illustrated with reference to the following Referential Examples 1-2.

Referential Example 1:

A microorganism, Pseudomonas BMG 13-A7 strain (deposited in an authorized Japanese depository "Fermentation Research Institute" under the accessible number FERM-P 5083), which had been cultured on an agar slant medium, was inoculated in a 500-mℓ Erlenmeyer flask which contained 110 mℓ of such a liquid medium (pH 7.4) comprising 1.5% of maltose, 0.3% of yeast extract, 1.0% of N.Z.-amine (type: A; product of FUMCO-Shefield Chemical Corp.). It was cultured at 27°C for 24 hours on a rotary shaker (180 revolutions per minute) to obtain a seed culture.

The seed culture (110 mℓ) was then inoculated to a 30-ℓ jar-fermentor made of stainless steel and containing 15 ℓ of the same liquid medium as that described above. The microbial strain was then cultured at 27°C for 40 hours (stirring: 250 turns per minutes; aeration: 15 ℓ per minute). Cultures from two jars-fermentors were combined, and the microbial cells were then removed by a continuous centrifugal machine (10,000 revolutions per minute) to give 27 ℓ of the broth filtrate as a supernatant (potency: 52 u./mℓ). The supernatant was charged into a column (internal diameter: 65 mm) of 1.5 ℓ of "Amberlite XAD-2". After washing the column with 5 ℓ of water, the column was eluted with a (1:1) mixture of methanol and water, and the eluate was collected in 17.5 mℓ-fractions. Fraction Nos. 52-295 were combined together and then concentrated to dryness under reduced pressure, thereby to afford 13.4 g of a crude powder of bactobolin (potency; 71 u./mg).
Yield: 68.0%.

Referential Example 2:

In 20 mℓ of water was dissolved 13.4 g of the crude bactobolin powder obtained in Referential Example 1. The resultant solution was charged into a column (internal diameter: 33 mm) of 600 mℓ of "Amberlite CG-50 Type I" {(7:3) mixture of the $NH^+$ form and $H^+$ form}. The column was thereafter developed with water and the eluate was were collected in 17 mℓ-fractions. Fraction Nos. 127-280 were combined together and then concentrated to dryness under reduced pressure, thereby obtaining 1.7 g of pale yellow powder of bactobolin (potency: 489 u./mg).
Yield: 91.1%.

The above powder was thereafter dissolved in 4 mℓ of water, and the resultant solution was again charged into a column (internal diameter: 14 mm) of 100 m of "Amberlite CG-59 Type I" {(7:3) mixture of the $NH^+$ form and $H^+$ form}. The column was developed with water and the eluate was collected in 14 mℓ-fractions. Fraction Nos. 31-48 were combined and then concentrated to dryness under reduced pressure to obtain 940 mg of bactobolin as pure, white powder (potency: 719 u./mg).
Yield: 77.3%.

Certain exemplary preparations of the agent of this invention will next be described.

Preparation Example 1:

An aqueous solution of bactobolin was asceptically filled in vials so that 1 mg (potency) of bactobolin was in each vial.

Preparation Example 2: Bactobolin 10 parts by weight
Lactose 60 part by weight
Magnesium stearate 5 parts by weight

Those ingredients were mixed together evenly and then filled in capsules to give 10 mg (potency) of bactobolin per capsule.

Preparation Example 3: Olive oil 160 parts by weight
Polyoxyethylene lauryl ether 10 parts by weight
Sodium hexametaphosphate 5 parts by weight

Fifty parts of bactobolin were added to and mixed uniformly with a uniform base composed of the above-mentioned ingredients. The resultant mixture was filled in soft capsules for rectal administration to give 50 mg (potency) of bactobolin per capsule.

Experimental allergic encephalomyelitis (EAE) is autoimmune encephalomyelitis which is induced upon challenge by an antigen to the medullary sheath of the central nerve, and it is considered to be a good animal model of multiple sclerosis {Paterson, P.Y. "Autoimmunity: Genetic Immunologic Virologic and Clinical Aspects" Academic Press, New York (1977); and Alvord, E.C. Jr.. Kies, M.W. and Suckling, A.J. "Experimental Allergic Encephalomyelitis - A Useful Model for Multiple Sclerosis" Alan R. Liss, New York (1984)}.

The prophylactic or therapeutic effects of bactobolin or autoimmune diseases will be demonstrated by the following Tests:

Test 1:

Suppressive Effects of bactobolin on Acute Experimental Autoimmune Encephalomyelitis (EAE) of Rat in Induction Phase

To animals (rats) as sensitized with myelin basic protein/complete Freund adjuvant (hereinafter affreviated as MBP/CFA) for immunization, 0.5 mg/kg of bactobolin was administered by intraperitoneal injection daily from the next day (Day 1) of the immunization to Day 8. This administration period corresponds approximately to the induction phase of EAE. The results obtained showed that all the control rats (receiving no bactobolin) developed EAE within the period of the study, whereas none of the rats developed EAE in the bactobolin-administered group (see Table 1 below).

It has also been demonstrated that bactobolin suppresses histologic EAE markedly and acts suppressively against the induction phase of EAE (see Table 2 below).

**Table 1**

Effects of Bactobolin on Clinical Signs of EAE
(the treatment made during the inductive phase)

| Compound tested | Dose (mg/Kg) | Administration period | Incidence of EAE: Number of animals affected/Number of animals tested | Paralysis* score (±SD) |
|---|---|---|---|---|
| Control** (No bactobolin administered) | - | - | 5/5 | 10.2 ± 1.9 |
| Bactobolin | 0.5 | 1-8 days | 0/5 | 0 ± 0 |

The animals were observed for 16 days after their immunization, followed by pathological examination of the sacrificed rats.

* Paralysis score showed a sum of the scores daily estimated during the disease.

** The control rats were injected with 0.5 mℓ of a phosphate buffer solution (PBS) daily.

**Table 2**

Effects of Bactobolin on Histologic EAE (the treatment made during the inductive phase)

| Compound tested | Number of animals tested | Histologic score* (±SD) |
|---|---|---|
| Control (No bactobolin administered) | 6 | 3.0 ± 0.3 |
| Bactobolin | 5 | 0.5 ± 0.6 |

* Histologic score is the mean of those for six separate sections, including the spinal cord and brain.

The histologic score was estimated in five ratings of from zero to 4 .

Test 2:

Suppressive Effects of bactobolin on EAE in Induction Phase

Bactobolin was administered intraperitoneally to the MBP CFA-immunized animals (rats) during a period (Day 8-14), which was considered to correspond primarily to the effector phase of EAE. and during this period the effects of bactobolin was estimated. Bactobolin also suppressed the incidence of EAE markedly in the present experiment (see Table 3 below).

In addition, the administration of bactobolin during the period of Day 8-14 also suppressed histologic lesion markedly (see Table 4 below).

## Table 3

Effects of Bactobolin on Clinical Signs of EAE (the treatment made during the effector phase, Day 8-14 after the immunization)

| Compound tested | Dose (mg/Kg) | Incidence of EAE (number of animals affected number of animals tested) | Paralysis score* (±SD) |
|---|---|---|---|
| Control (No bactobolin administered) | - | 5/5 | 13.8 ± 2.0 |
| Bactobolin | 0.5 | 0/5 | 0 ± 0 |

* The animal were sacrified for pathological study on Day 15 P.I.

## Table 4

Effects of Bactobolin on Histologic EAE (the treatment made during the effector phase)

| Compound tested | Number of animals tested | Histologic score* (±SD) |
|---|---|---|
| Control (No bactobolin administered) | 5 | 2.4 ± 0.5 |
| Bactobolin | 5 | 0.1 ± 0.1 |

* The histopathology of the treated animals was evaluated for 15 days after the immunization.

Test 3:

Suppressive Effects of bactobolin given after Incidence of EAE

A study was conducted to determine whether the intraperitoneal administration of bactobolin made after the incidence of EAE was therapeutically effective, or not. The administration of bactobolin was started on the day of the EAE incidence when the first clinical sign was noted in each case. As a result, no substantial influences were observed depending on the time of administration of bactobolin during the duration of illness but the paralysis score was suppressed significantly (see Table 5 below).

Table 5

Effects of Bactobolin on EAE after Onset of Clinical Signs*

| Compound tested | Dose (mg/Kg) | Whole duration of illness (days ± SD) | Paralysis score* (±SD) |
|---|---|---|---|
| Control (no bactobolin administered) | - | 5.0 ± 1.1 | 11.2 ± 1.9 |
| Bactobolin | 0.5 | 4.4 ± 1.0 | 4.6 ± 1.2 |

* The treatment was started on the day when the first clinical sign was noted in each case.
{The control group was administered with 0.5 mℓ of a phosphate buffer solution (PBS) only daily after clinical onset of EAE}.

Test 4:

Effects of bactobolin on Passively Induced EAE

In order to further clarify the suppressive effects of bactobolin on EAE during the effector phase, its suppressive effects on passively induced EAE were investigated. Bactobolin also suppressed the incidence of passively induced EAE markedly (see Table 6 below).

Table 6

Effects of Bactobolin on Passively Induced EAE

| Treatment of recipients | Dose (mg/Kg) | Incidence of EAE (days ± SD) | Paralysis score ( ± SD) |
|---|---|---|---|
| Control (no bactobolin but PBS administered) | - | 2/2 | 9.5 ± 0.5 |
| Bactobolin | 0.5 | 0/2 | 0 ± 0 |

Note 1:-Every recipient was transferred with $2 \times 10^7$ precultured spleen cells obtained from rats immunized with GPBP/CFA, 11 days before. Here, GPBP means guinea pig myeline basic protein; and CFA means complete Freund's adjuvant.

Note 2:-The recipients were administered from Day 1 to Day 8 P.I., and sacrificed on Day 9 P.I.

Test 5:

Suppressive Effects of bactobolin on Lymphocyte Proliferative Responses

Using lymph node cells which were separately collected separately from control rats and from the bactobolin-intraperitoneally administered rats on Day 11 (the 11th day after the sensitization), the lymphocyte proliferative responses to specific antigen, as well as the response to Con A were assessed. No lymphocyte proliferative responses to specific antigen were observed with the lymph node cells of the bactobolin-administered group. The response to Con A was also suppressed with the lymph node cells of the bactobolin-administered group (see Table 7 below).

## Table 7

| Antigen or mitogen | Control group (no bactobolin administered) | | Bactobolin-administered group | |
|---|---|---|---|---|
| | CPM ± SD | | CPM ± SD | |
| - | 11,927 | 531 | 5,858 | 316 |
| MBP | 30,214 | 2,081 | 2,507 | 670 |
| PPD | 54,554 | 2,958 | 5,315 | 696 |
| Con A | 338,783 | 6,433 | 32,271 | 27,295 |

Note 1:-Animals belonging to Bactobolin-administered group were daily intraperitoneally with 0.5mg/kg of bactobolin from Day 1 P.I. to Day 8 P.I., and the control animals were treated with PBS.

Note 2:-Lymph node cells were obtained from control and bactobolin-treated rats 11 days after the sensitization. These lymphoid cells were cultured with GPBP (10 µg/mℓ), PPD (10 µg/mℓ) or Con A (2 µg/mℓ) for 3 days. The proliferative responses were assessed by measuring the uptake of tritiated thymidine.

In the above, CPM means the measured number of count per minute; MBP means myelin basic protein; PPD means a purified protein derivative; and Con A means concanavalin A.

As demonstrated in the above Tests, bactobolin significantly suppressed EAE in both the induction phase and effector phase so that the prophylactic effects of bactobolin on EAE have been recognized. Bactobolin also exhibited suppressive effects on EAE after its incidence, whereby its therapeutic effects have also been recognized.

## Claims

1. A prophylactic or therapeutic agent for autoimmune diseases, comprising bactobolin of the formula:

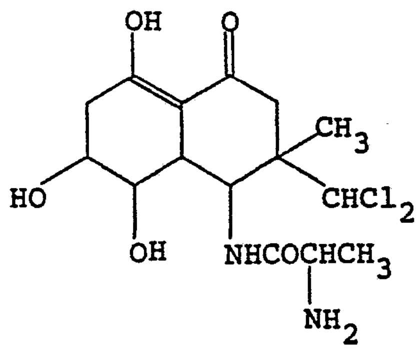

(I)

or a pharmaceutically acceptable acid addition salt of bactobolin as the active ingredient, in association with a pharmaceutically acceptable carrier for the active ingredient.

2. A method of prophylactically or therapeutically treating an autoimmune disease, which comprises administering an effective amount of bactobolin or a pharmaceutically acceptable acid addition salt of bactobolin to an animal having suffered from the autoimmune disease.

3. A method of suppressing the immune response in a living animal, including human, which comprises administering an effective amount of bactobolin or a pharmaceutically acceptable acid addition salt of bactobolin to a living animal where the immune response is to be suppressed.

4. Use of bactobolin as an immunosuppressive agent or for the production of an immunosuppressive agent.

5. A method of suppressing the immune response in a living animal, which comprises administering an effective amount of bactobolin to a living animal where the immune response are to be suppressed.

6. A method for suppressing prophylactically or therepeutically treating an experimental allergic encephalomyelitis in the induction phase or in the effector phase, which comprises administering an effective amount of bactobolin to an animal having suffered from the experimental allergic encephalomyelitis as induced.

7. A method for suppressing lymphocyte proliferative response to antigen or mitogen, which comprises administering an effective amount of bactobolin to a living animal where the lymphocyte proliferative response to antigen or mitogen is to be suppressed.

8. Use of bactobolin of the formula

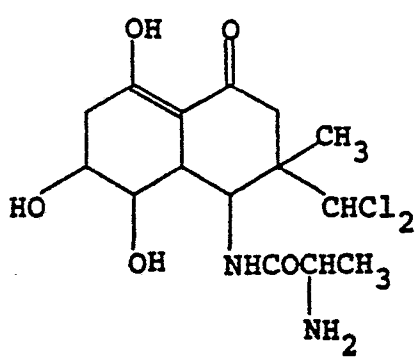

(I)

for preparing a prophylactic or therapeutic agent for autoimmune diseases.

9. Use of bactobolin of the formula

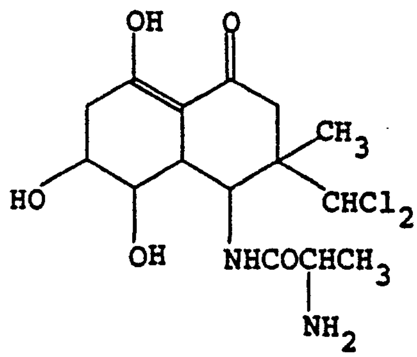

(I)

for preparing an agent for suppressing prophylactically or therapeutically treating an experimental allergic encephalomyelitis.